# EUROPEAN PATENT APPLICATION

(11) **EP 4 371 559 A1**
(43) Date of publication of application: **22.05.2024**
(21) Application number: 22854852.5
(22) Date of filing: 18.01.2022
(51) Int. Cl.: A61K 9/22, A61K 9/30, A61K 31/4422, A61K 47/12, A61P 9/12

(54) **OSMOTIC PUMP CONTROLLED-RELEASE TABLET OF INSOLUBLE DRUG AND PREPARATION METHOD THEREFOR**

(30) Priority: 09.08.2021 CN 202110906250
(71) Applicant: Beijing Wehand-Bio Pharmaceutical Co., Ltd, Beijing 102600 (CN)
(72) Inventor: LIU, Yuling, Beijing 102600 (CN); WANG, Hongliang, Beijing 102600 (CN); LIU, Zhihua, Beijing 102600 (CN); SHENG, Wei, Beijing 102600 (CN); CHEN, Luxiao, Beijing 102600 (CN); XU, Xueqing, Beijing 102600 (CN); CHEN, Yankun, Beijing 102600 (CN); MA, Rui, Beijing 102600 (CN)
(74) Representative: Bandpay & Greuter
(86) International application number: PCT/CN2022/072568
(87) International publication number: WO 2023/015847

(57) **Abstract**

The present invention relates to an osmotic pump controlled-release tablet of an insoluble drug and a preparation method therefor. The osmotic pump controlled-release tablet comprises a tablet core, a semi-permeable membrane coating and a drug-release hole, wherein the tablet core comprises a solid dispersion of the insoluble drug and a penetration enhancer, the solid dispersion of the insoluble drug comprises the insoluble drug and a carrier, the insoluble drug is selected from nicardipine, nifedipine and felodipine and pharmaceutically acceptable salts thereof, and the carrier is selected from organic acids. Compared with the prior art, the drug solubility of the osmotic pump controlled-release tablet of the insoluble drug of the present invention is greatly improved, a large amount of penetration enhancers or other excipients do not need to be used in the tablet core, the drug-loading capacity is improved, and the tablet core is not too large; the degree of release in vivo is greatly improved; and the preparation process is simple, has low requirements for equipment and a low cost, and facilitates industrialized production.

## Description

### Technical Field

The present disclosure relates to the field of pharmaceutical formulations. Specifically, the present disclosure relates to an osmotic pump controlled-release tablet of an insoluble drug and a preparation method therefor.

### Background Art

Sustained- and controlled-release formulations can greatly reduce the number of times patients take medicines, reduce fluctuations in plasma concentration, reduce the discomfort caused by the peak-valley phenomenon, and reduce toxic and side effects, while improving patient compliance. Osmotic pump controlled-release formulations are praised as the most desirable oral sustained-/controlled-release dosage form due to their ability to release drugs sustainably and stably at a constant rate without being subject to gastrointestinal variables such as peristalsis, the pH value, and the gastric emptying time. Depending upon the solubility characteristics of different drugs, the osmotic pump may be divided into a single-chamber osmotic pump, a push-pull osmotic pump (a multi-chamber osmotic pump), a microporous membrane osmotic pump, etc. Of these, the single-chamber osmotic pump is suitable for watersoluble drugs (5% to 10%), which has the advantages of a simple preparation process, a stable drug release rate and the like, but it has high requirements for drug solubility, so there are currently very few products on the market. Insoluble drugs are generally designed as multi-chamber osmotic pumps including a drug-containing tablet core and a push-pull layer to increase the drug release power. For example, the representative product "nifedipine osmotic pump tablets" marketed early is a multi-chamber osmotic pump tablet, but this dosage form is prepared by a complex preparation process, which requires multiple times of tabletting and laser identification and perforation and has extremely high requirements for auxiliaries and process equipment, making industrialization difficult. Therefore, how to effectively improve the solubility of insoluble drugs and develop them into single-chamber osmotic pump drug delivery systems is of great significance.

As a result of solubility, the cumulative release characteristics of an insoluble drug, if prepared directly into a single-chamber osmotic pump, are very low (Study on Nicardipine Hydrochloride of Controlled-Release Tablets, ZHENG Qilan, Shenyang Pharmaceutical University, Master Thesis). Therefore, drug solubilization and penetration enhancing means, *e.g*. techniques such as solid dispersions and cyclodextrin inclusion, are often taken to improve the solubility of insoluble drugs, and furthermore, a suitable penetration enhancer is selected to increase the indoor osmotic pressure and viscosity, so as to realize development of single-chamber osmotic pump formulations of insoluble drugs. In current researches, the solubilization techniques such as solid dispersion and cyclodextrin inclusion often use polymer carrier materials such as PVPK30, PEG, poloxamer, and cyclodextrin in an amount of about 1 to 5 times the dose of the drug, and after prepared into an intermediate, a penetration enhancer, a filler and the like need to be further added to be pressed into a tablet core and then coated to obtain a osmotic pump tablet. For example, some scholars have prepared baicalein and PVPK30 at a weight ratio of 1:4 into a solid dispersion, and added a penetration enhancer NaCl and a suspending agent CMC-Na to prepare a single-layer osmotic pump formulation having cumulative release characteristics of greater than 90% (Preparation of single-layer osmotic pump controlled release tablets of curcumin solid dispersions and formulation optimization, YAN Wei, HU Chunxia, ZHANG Zhiqiang, Chinese Traditional Patent Medicine, 2019, 1768-1772). For another example, some scholars have adopted the inclusion technology to prepare baicalein into an inclusion, in which the weight ratio of baicalein to dimethyl-β-cyclodextrin is 1:5, and to further add a penetration enhancer NaCl and a suspending agent CMC-Na to prepare a single-layer osmotic pump having cumulative release characteristics of 80% or more (Preparation technique of monolithic osmotic pump tablet containing inclusion complex of baicalein, ZHENG Xiangtao, HAO Haijun, HAN Ru et al., Academic Journal of Second Military Medical University, 2015, 513-517). However, a large amount of functional auxiliaries added for the sake of improving the drug solubility will render the tablet core too heavy, which is not conducive to subsequent coating and administration after marketing. Moreover, the dose of drug is larger in an osmotic pump formulation than in a general formulation, so it is less practical to use excessive auxiliaries. It is thus critical for process feasibility of formulations to control the amount of the functional auxiliaries while improving the drug solubility.

Although it has been reported in the early days that single-chamber osmotic pumps prepared directly from an insoluble drug, a suspending agent, and a penetrating agent at a certain ratio achieve better release effects, it is unclear whether it is suitable for specific drug crystal forms. Meanwhile, the present inventors have found in their earlier studies that organic acids may have good solubilizing and penetration-enhancing effects on some insoluble drugs. An organic acid is used as a penetration enhancer to prepare a tablet core containing an insoluble drug and a simply single-layer osmotic pump technology is adopted; as a result, the release characteristics of the drug in water are significantly improved and the drug release over 24 h reaches 90% or more (Patent No.: ZL200510065906.2: Preparation of Nicardipine Hydrochloride Monolithic Osmotic Pump Tablets and Its *in Vitro* Release Behavior, MA Rui, WANG Hongliang, LIU Yuling et al., 2011, China Pharmacy, 1967-1969). However, for some drugs or drug crystal forms, their solubility can still not be effectively improved according to the above method, but can only resort to more complicated technologies such as double-layer osmotic pump formulations, in which a booster layer is added and a large amount of functional auxiliaries are required, increasing the weight of the tablet core and thus increasing the difficulties of the formula and preparation process of the formulation. Therefore, there is a need for a more appropriate method to further improve the drug solubility.

### Summary of Invention

In view of the above problems, the present inventors have unexpectedly discovered in their researches that after insoluble drugs are prepared into solid dispersions using organic acids as carriers, the solubility of the drugs in the solutions of the organic acids is further improved. In addition, compared with use of polymer carrier materials, use of organic acids as carriers can significantly reduce the amounts of the carrier materials, which is more conducive to controlling the amount of the excipient in the formulation and improving the process feasibility.

In view of the foregoing, according one aspect of the present disclosure, there is provided an osmotic pump controlled-release tablet of an insoluble drug, wherein osmotic pump controlled-release tablet comprises a tablet core, a semi-permeable membrane coating, and a drug-release hole, the tablet core comprises a solid dispersion of the insoluble drug and a penetration enhancer, the solid dispersion of the insoluble drug comprises an insoluble drug and a carrier;
the insoluble drug is selected from the group consisting of nicardipine, nifedipine, felodipine, and pharmaceutically acceptable salts thereof, and the carrier is selected from the group consisting of organic acids. The solubility of the osmotic pump controlled-release tablet thereby obtained is greatly improved without a need to use a large amount of penetration enhancers or other excipients in the tablet, the drug-loading capacity is improved, and the tablet core is not too large; the *in vivo* release characteristics are greatly improved; the preparation process is simple, the requirements for equipment and the costs are low, and it is easy to be industrialized.

The osmotic pump controlled-release tablet according to the present disclosure comprises a plurality of osmotic pump controlled-release tablets such as a single layer, a double layer or a multiple layer.

According to some examples of the present disclosure, the penetration enhancer is an organic acid or a combination of an organic acid with any one or more of sodium chloride, mannitol, and lactose. Through extensive experimental research, the present inventors have found that using organic acids as the penetration enhancer can greatly improve the drug solubility. However, if the solubility of some drugs is too high, this may cause the drugs to be released too fast and thus reduce the medication effect. In some examples, selecting organic acids or combinations of organic acids with any one or more of sodium chloride, mannitol, and lactose as penetration enhancers may control the release rate of the drug within an appropriate range to fulfill the objective of controlled release.

According to some examples of the present disclosure, the organic acids in the carrier are citric acid, fumaric acid, succinic acid, tartaric acid, cholic acid, lecithin or deoxycholic acid, preferably citric acid, fumaric acid or succinic acid, further preferably citric acid.

According to some examples of the present disclosure, the organic acids in the penetration enhancer are citric acid, fumaric acid, succinic acid, tartaric acid, cholic acid, lecithin or deoxycholic acid, preferably citric acid, fumaric acid or succinic acid, further preferably citric acid.

According to some examples of the present disclosure, the organic acids in the carrier and the organic acids in the penetration enhancer are the same kind of organic acids. For example, the organic acids in the carrier and in the penetration enhancer are both citric acid, or optionally the organic acids in the carrier and in the penetration enhancer are different kinds of organic acids.

According to some examples of the present disclosure, the insoluble drug according to the present disclosure is nicardipine hydrochloride, further preferably α-crystal-form nicardipine, β-crystal-form nicardipine or a mixed crystal thereof.

According to some examples of the present disclosure, the weight ratio of the insoluble drug to the carrier in the solid dispersion is 1:(0.1-1), or 1:(0.1-0.8), or 1:(0.1-0.6), or 1:(0.1-0.4), or 1:(0.1-0.3), or 1:(0.1-0.25), or 1:(0.1-0.2); or 1:(0.15-1), or 1:(0.15-0.8), or 1:(0.15-0.6), or 1:(0.15-0.4), or 1:(0.15-0.3), or 1:(0.15-0.25); or 1:(0.19-0.6); or 1:(0.19-0.56); or 1:(0.2-0.56); or 1:(0.2-1), or 1:(0.2-0.8), or 1:(0.2-0.6), or 1:(0.2-0.4), or 1:(0.2-0.3), or 1:(0.2-0.25); or 1:(0.25-1), or 1:(0.25-0.8), or 1:(0.25-0.6), or 1:(0.25-0.4), or 1:(0.25-0.3); or 1:(0.3-1), or 1:(0.3-0.8), or 1:(0.3-0.6), or 1:(0.3-0.4). The present inventors have found through extensive experiments that by using a smaller carrier weight as described above, the present disclosure can significantly improve the solubility of insoluble drugs, and thus the amount of the excipient in the tablet can be greatly reduced.

According to some examples of the present disclosure, the penetration enhancer is an organic acid; preferably, the organic acid is citric acid. According to some examples of the present disclosure, the penetration enhancer accounts for 10% to 70%, preferably 20% to 60% of the total weight of the tablet core.

In the osmotic pump controlled-release tablet of the insoluble drug according to the present disclosure, the tablet core may further comprise an excipient selected from the group consisting of, *e.g.* a filler, a disintegrant, a diluent, an adhesive, and a lubricant.

According to some examples of the present disclosure, the semi-permeable membrane coating accounts for 5% to 12%, preferably 5% to 10% of the weight of the tablet.

According to some examples of the present disclosure, the semi-permeable membrane coating is composed of a film-forming material and a plasticizer, and preferably, the weight ratio of the film-forming material to the plasticizer is 9 to 99: 1.

According to another aspect of the present disclosure, there is provided a solid dispersion of an insoluble drug, comprising an insoluble drug and a carrier material, wherein the insoluble drug is selected from the group consisting of nicardipine, nifedipine, felodipine, and pharmaceutically acceptable salts thereof, and the carrier material is selected from the group consisting of organic acids;
optionally, the organic acids are citric acid, fumaric acid, succinic acid, tartaric acid, cholic acid, lecithin or deoxycholic acid; preferably, citric acid, fumaric acid or succinic acid, further preferably citric acid.

According to another aspect of the present disclosure, there is provided a method for preparing an osmotic pump controlled-release tablet of an insoluble drug, comprising steps of:
a) preparing a solid dispersion of the insoluble drug by a solvent method;
b) mixing the solid dispersion with a penetration enhancer, and optionally adding an appropriate amount of an additional excipient;
c) directly tablet pressing or pressing to form a tablet core after granulation; and
d) externally covering with a semi-permeable membrane and perforating by laser or mechanically.

Preferably, the step of the solvent method comprises: dissolving the drug and a carrier in an organic solvent, removing the organic solvent under reduced pressure until foaming, and subjecting to rotary evaporation, sieving, and vacuum drying to obtain the solid dispersion of the insoluble drug,
preferably, the organic solvent is selected from the group consisting of ethanol, methanol, and tert-butanol, more preferably methanol.

Optionally, the step of the solvent method comprises: dissolving the drug and a carrier in an organic solvent, removing the organic solvent by spray drying, and subjecting to vacuum drying to obtain the solid dispersion of the insoluble drug, wherein the organic solvent is selected from the group consisting of ethanol, methanol, and tert-butanol, more preferably methanol.

Optionally, the step of the solvent method comprises: dissolving the drug and a carrier in an organic solvent, removing the organic solvent by a freeze drying method to obtain the solid dispersion of the insoluble drug, wherein the organic solve is tert-butanol.

The preparation process for the osmotic pump tablet provided in the present disclosure allows an insoluble drug and organic acids to form a solid dispersion, prepares it into a tablet core by adding an organic acid and an additional appropriate excipient, and further processes it to obtain the osmotic pump controlled-release tablet. In the formula, the insoluble drug and the organic acids are prepared into a solid dispersion, and at the same time an organic acid is used as a penetration enhancer, which can greatly improve the solubility of the insoluble drug, while controlling the amounts of the excipients such as the carrier and the penetration enhancer at lower levels, overcoming the problem about an insufficient drug-loading capacity or too large tablet cores. Meanwhile, the preparation process is simple and easy to do, with the advantages of, *e.g.* good quality controllability, low equipment requirements and ease to be industrialized.

### Description of Drawings

FIG. 1 is an XRD pattern of Nic (nicardipine hydrochloride) drug; FIG. 1A: an XRD pattern of α-Nic; FIG. 1B: an XRD pattern of β-Nic.
FIG. 2 is an XRD pattern of Nic/citric acid (w/w = 1:0.37) solid dispersion; FIG. 2A: an XRD pattern of α-Nic/citric acid solid dispersion, FIG. 2B: an XRD pattern of β-Nic/citric acid solid dispersion.
FIG. 3 is an XRD pattern of β-Nic/PVPK30 solid dispersion; a: an XRD pattern of β-Nic; b: an XRD pattern of PVPK30; c: an XRD pattern of β-Nic/PVPK30 (w/w = 1:1) physical mixture; d: an XRD pattern of β-Nic/PVPK30 (w/w = 1:3) physical mixture; e: an XRD pattern of β-Nic/PVPK30 (w/w = 1:5) physical mixture; f: an XRD pattern of β-Nic/PVPK30 (w/w = 1:1) solid dispersion; g: an XRD pattern of β-Nic/PVPK30 (w/w = 1:3) solid dispersion; h: an XRD pattern of β-Nic/PVPK30 (w/w = 1:5) solid dispersion.
FIG. 4 is an XRD pattern of β-Nic/PEG6000 solid dispersion; a: beta crystal form of Nic; b: an XRD pattern of PEG6000; c: an XRD pattern of β-Nic/PEG6000 (w/w = 1:1) physical mixture; d: an XRD pattern of β-Nic/PEG6000 (w/w1:3) physical mixture; e: an XRD pattern of β-Nic/PEG6000 (w/w = 1:5) physical mixture; f: an XRD pattern of β-Nic/PEG6000 (w/w1:1) solid dispersion; g: an XRD pattern of β-Nic/PEG6000 (w/w = 1:3) solid dispersion; h: an XRD pattern of β-Nic/PEG6000 (w/w1:5) solid dispersion.
FIG. 5 is an XRD pattern of β-Nic/SDF68 solid dispersion; a: an XRD pattern of β-form Nic; b: an XRD pattern of SDF68; c: an XRD pattern of β-Nic/SDF68 (w/w = 1:1) physical mixture; d: an XRD pattern of β-Nic/SDF68 (w/w = 1:3) physical mixture; e: an XRD pattern of β-Nic/SDF68 (w/w = 1:5) physical mixture; f: an XRD pattern of β-Nic/SDF68 (w/w = 1:1) solid dispersion; g: an XRD pattern of β-Nic/SDF68 (w/w = 1:3) solid dispersion; h: an XRD pattern of β-Nic/SDF68 (w/w = 1:5) solid dispersion.
FIG. 6 is a profile of release of the Nic osmotic pump tablet of the present disclosure and a perdipine sustained-release capsule.
FIG. 7 is a profile of release of single-layer osmotic pump tablets of Nic solid dispersions in which different penetration enhancers are used.
FIG. 8 is a profile of release of osmotic pump tablets of Nic solid dispersions in which different combinations of penetration enhancers are used.
FIG. 9 is a profile of release of osmotic pump tablets of Nic solid dispersions prepared at different ratios of citric acid to penetration enhancers.
FIG. 10 is a profile of release of osmotic pump tablets of Nic solid dispersions made up of different coatings.
FIG. 11 is a profile of release of osmotic pump tablets of Nic solid dispersions with different weight gains of coatings.
FIG. 12 is a drug-time profile of the plasma concentrations of the Nic osmotic pump tablet of the present disclosure and the perdipine sustained-release capsule in beagle.
FIG. 13 is a profile of release of osmotic pump tablets prepared from nicardipine hydrochloride as a raw material and from a nicardipine hydrochloride solid dispersion as a raw material.

### DETAILED DESCRIPTION

The present disclosure will be further described in detail below with reference to the drawings and examples. From those exemplary descriptions, the features and advantages of the present disclosure will become clearer. Any example illustrated herein for an exemplary purpose is not necessarily construed to be superior to or better than the other examples.

### Example 1 Preparation and XRD analysis of solid dispersion of nicardipine hydrochloride (Nic)

### Preparation of solid dispersion

Nicardipine hydrochloride was used as a model drug, and an organic acid (including succinic acid, fumaric acid, and citric acid), PVPK30, PEG6000, and Pluronic F68 were selected as carrier materials to prepare solid dispersions by a solvent method (the carrier materials were an organic acid and PVPK30) and a solvent-melting method (the carrier materials were PEG6000 and Pluronic F68). The Nic solid dispersion was prepared by the following method:
Solvent method: Nicardipine hydrochloride as a pharmaceutical active ingredient and carrier materials were weighed according to the equivalents. 5 to 20 Equivalents of methanol were added. The organic solvent was removed under reduced pressure until foaming. The mixture was continued to be subjected to rotary evaporation (water bath at 40°C, 60 rpm) for 3 h, screened with 100 mesh sieve, and vacuum dried at 40°C for 12 h to obtain the Nic solid dispersion.
Solvent-melting method: The drug was weighed and dissolved in 5 to 20 equivalents of methanol. The carrier materials were melted in a water bath in proportion. The drug soup was added to the carrier solution, stirred rapidly, dried by evaporation to remove methanol, cured at - 20°C, and vacuum dried at room temperature for 48 h. The solid was dried and ground to obtain the Nic solid dispersion.

### XRD analysis of solid dispersion

Appropriate amounts of beta or alpha crystal form of Nic and a solid dispersion of Nic as well as the corresponding physical mixture thereof were weighed respectively and analyzed by XRD to observe the diffraction peaks of crystals. Detection conditions were as follows: Cu-Kα radiation source, graphite monochromator, measured pipe pressure: 40 kV, pipe current: 200 mA, diffraction range: 3°< 2θ < 60°, step: 0.02, residence time per step: 0.2 s.

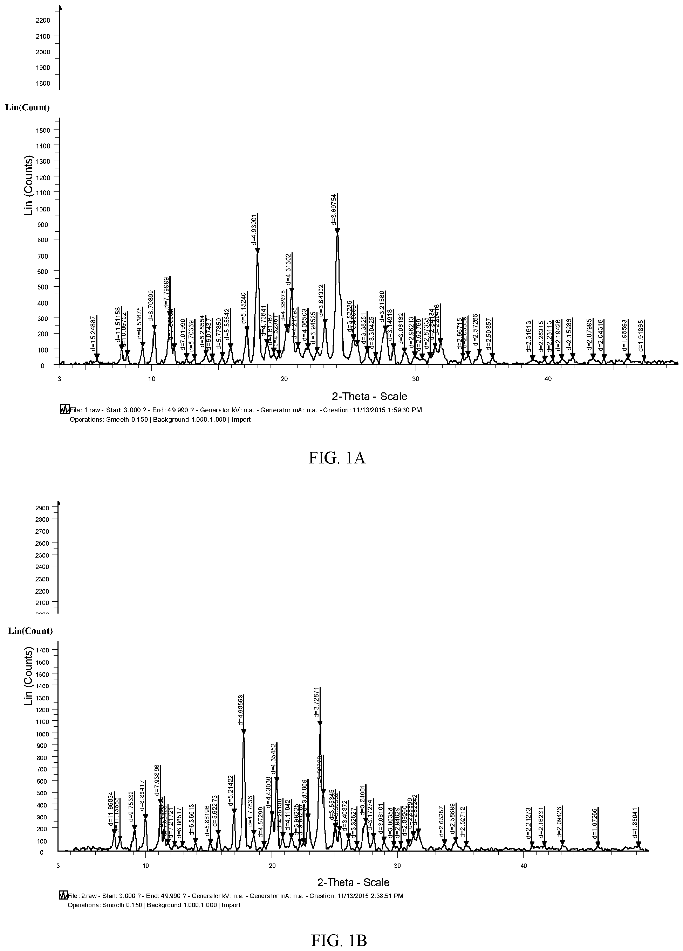

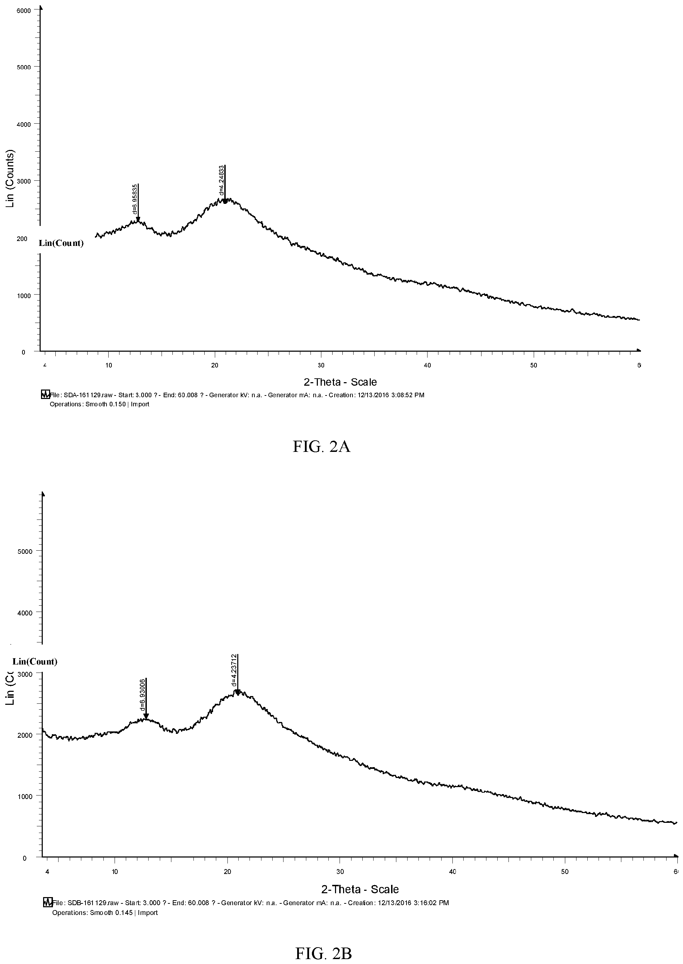

The results of FIGS. 1 and 2 showed that Nic (α, β crystal form) exhibited many characteristic crystal diffraction spikes, indicating that Nic was in a crystalline state. However, in the solid dispersion formed by Nic and carriers, the crystal diffraction peaks of the drug completely disappeared, suggesting that the crystal characteristics of Nic itself were inhibited and existed in an amorphous state after the drug and carriers formed a solid dispersion.

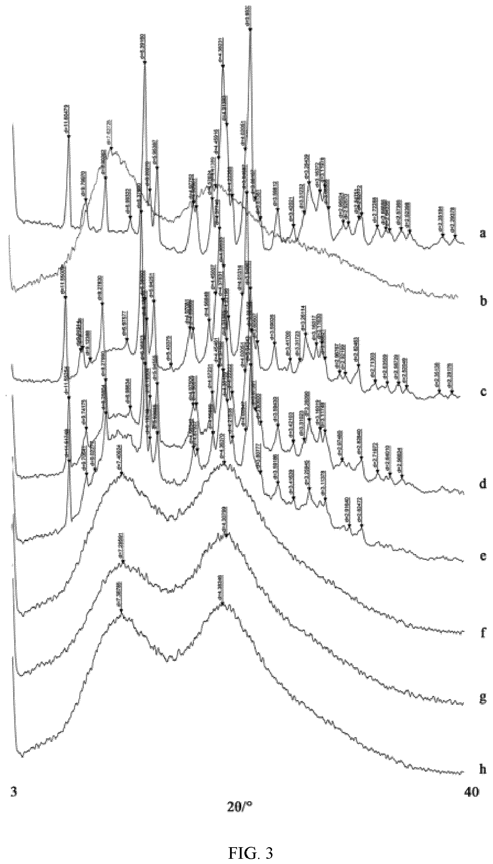

The result of FIG. 3 showed that Nic exhibited many characteristic crystal diffraction spikes, indicating that Nic was in a crystalline state and the carrier material PVP K30 was in an amorphous state. The crystal diffraction peaks of the drug in the physical mixture were visible, and there were no new peaks, indicating that the drug and the carrier materials were only mixed physically. However, in the solid dispersions formed by the drug and the carrier material PVPK30 at different dosage ratios, the crystal diffraction peaks of the drug completely disappeared, the drug exhibited similar characteristic peaks to those of the carrier material, and the crystalline characteristics of Nic itself were inhibited and existed in an amorphous state, suggesting that the drug and PVPK30 could always form solid dispersions at the three ratios.

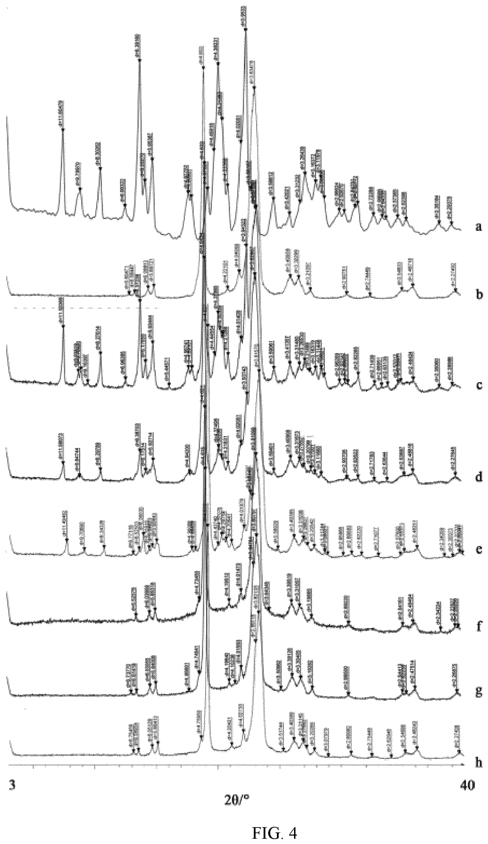

The result of FIG. 4 showed that Nic exhibited many characteristic crystal diffraction spikes, indicating that Nic was in a crystalline state and the carrier material PEG6000 appeared in a crystalline state. Both the crystal diffraction peaks of the drug and the characteristic peaks of the carrier material in the physical mixture were visible, and there were no new peaks. However, in the solid dispersions formed by the drug and the carriers at different dosage ratios, all the crystal diffraction peaks of the drug completely disappeared, and the drug exhibited similar characteristic peaks to those of the carrier material, which suggested that after the drug and the carriers formed solid dispersions, the crystalline characteristics of Nic itself were inhibited and existed in an amorphous state.

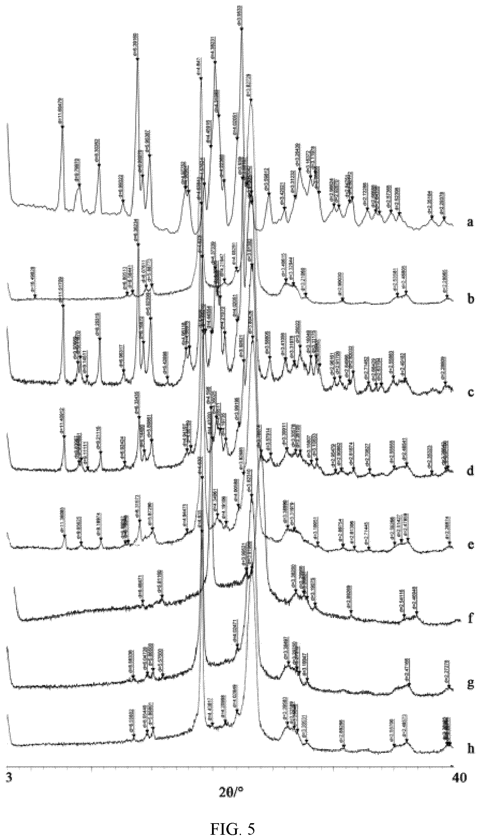

The result of FIG. 5 showed that Nic exhibited many characteristic crystal diffraction spikes, indicating that Nic was in a crystalline state and the carrier material SDF68 appeared in a crystalline state. Both the crystal diffraction peaks of the drug and the characteristic peaks of the carrier material in the physical mixture were visible, and there were no new peaks. However, in the solid dispersions formed by the drug and the carriers at different dosage ratios, all the crystalline diffraction peaks of the drug completely disappeared, and the drug exhibited similar characteristic peaks to those of the carrier material, which suggested that after the drug and the carriers formed solid dispersions, the crystalline characteristics of Nic itself were inhibited and existed in an amorphous state.

### Example 2 Determination of solubility of solid dispersion of nicardipine hydrochloride (Nic)

An appropriate amount (equivalent to 20 mg of drug dose) of the solid dispersion powder prepared in Example 1 was weighed separately, added to 1 mL of water or 1 mL of saturated citric acid solution, and immediately put in an air oscillator at 37°C and oscillated for 2 h. After the drug was dissolved, an equivalent dose of the solid dispersion powder was further added, and oscillated for 2 h. The above operations were repeated until an insoluble solid occurred in the solution. The solubility properties of the solid dispersions in water and in saturated citric acid at 37°C were determined, respectively.

**Table 1 Solubility of nicardipine hydrochloride drug and solid dispersion in water and organic acid solvent**

| Drug or Solid Dispersion | Drug-Loading Ratio (Weight Ratio) | Solubility in Water mg/ml (37°C) | Solubility of Saturated Citric Acid Solution mg/ml (37°C) |
|---|---|---|---|
| α-Form Nic | - | 7.2 | 46 |
| β-Form Nic | - | 11 | 50 |
| | 1:0.19 | 10.60 | 84.94 |
| β-Nic/citric acid physical mixture | 1:0.37 | 10.89 | 95.454 |
| | 1:0.56 | 11.23 | 93.20 |
| β-Nic/succinic acid solid dispersion | 1:0.23 | 31.24 | - |
| β-Nic/fumaric acid solid dispersion | 1:0.23 | 41.81 | - |
| α-Nic/citric acid solid dispersion | 1:0.37 | 130-150 | ≥500 |
| β-Nic/citric acid solid dispersion | 1:0.19 | 60.33 | ≥500 |
| β-Nic/citric acid solid dispersion | 1:0.28 | 50.28 | ≥500 |
| β-Nic/citric acid solid dispersion | 1:0.37 | 61 | ≥500 |
| β-Nic/citric acid solid dispersion | 1:0.56 | 49.82 | ≥500 |
| P-Nic/PVPK30 solid dispersion | 1:1 | - | 80-100 |
| P-Nic/PVPK30 solid dispersion | 1:3 | - | 100 |
| P-Nic/PVPK30 solid dispersion | 1:5 | - | 100 |
| β-Nic/PEG6000 solid dispersion | 1:1 | - | 60-80 |
| β-Nic/PEG6000 solid dispersion | 1:3 | - | 100-120 |
| β-Nic/PEG6000 solid dispersion | 1:5 | - | 100-120 |
| P-Nic/SDF68 solid dispersion | 1:1 | - | 40-60 |
| P-Nic/SDF68 solid dispersion | 1:3 | - | 80-100 |
| P-Nic/SDF68 solid dispersion | 1:5 | - | 120-140 |

The results showed that the solubility of the nicardipine hydrochloride solid dispersions prepared by different carriers in water or in citric acid was remarkably higher than the solubility of the drug itself in water or citric acid, and the solubility of the Nic/citric acid solid dispersions in citric acid was remarkably higher than the solubility of the Nic/polymer material solid dispersions in citric acid. The solid dispersion using an organic acid as a carrier had a higher drug-loading ratio.

### Example 3 Study on preparation solvents for solid dispersions

Beta-nicardipine hydrochloride was used as a model drug, citric acid was selected as a carrier material, and ethanol, 95% ethanol, ethyl acetate, dichloromethane, and methanol were used as solvents respectively to prepare Nic-citric acid solid dispersions according to the solvent method described above. The feasibility of preparation was studied, and the content of the residual solvent was determined by the gas chromatography. The results were as shown in Table 2 below.

**Table 2 Analysis results of the preparation solvents**

| Types of Solvent | β-Nic:citric acid=1:0.37 (w/w) Amount of Solvent | Preparation of Solid Dispersion | Determination of Residual Solvent |
|---|---|---|---|
| Anhydrous ethanol | 45 ml | Well dispersed, larger amount of solvent used | 0.31% |
| 95% Ethanol | 15 ml | Well dispersed, difficult for solvent to be dried by evaporation | 0.59% |
| Ethyl acetate | 50 ml of drug, basically insoluble | Failure to prepare | |
| Dichloromethane | 50 ml of carrier, basically insoluble; drug, dissolved | Failure to prepare | |
| Methanol | 15 ml | Well dispersed, high preparation efficiency | 0.07% |

### Example 4 Preparation and release detection of osmotic pump tablets

Nicardipine hydrochloride (β-form) and anhydrous citric acid were weighed at a drug-loading ratio (w/w) of 1:0.37 to prepare an Nic/citric acid solid dispersion according to the solvent method of Example 1. The osmotic pump tablet formulation 1 was prepared according to formula 1 listed in Table 3 below.

**Table 3 Composition of tablet core of formula 1**

| | |
|---|---|
| Nic/citric acid solid dispersion | 109.6 mg |
| Citric acid | 130.4 mg |
| Microcrystalline cellulose | 160 mg |
| PVPK30 | 6 mg |
| PVPP | 6 mg |
| Magnesium stearate | 3 mg |

Nicardipine hydrochloride (β-form) and anhydrous citric acid were weighed at a drug-loading ratio (w/w) of 1:0.19 to prepare an Nic/citric acid solid dispersion according to the solvent method of Example 1. The osmotic pump tablet formulation 2 was prepared according to formula 2 listed in Table 4 below.

**Table 4 Composition of tablet core of formula 2**

| | |
|---|---|
| Nic/citric acid solid dispersion | 95.2 mg |
| Citric acid | 144.8 mg |
| Microcrystalline cellulose | 160 mg |
| PVPK30 | 6 mg |
| PVPP | 6 mg |
| Magnesium stearate | 3 mg |

The composition and preparation method for the coatings of formulation 1 and formulation 2 were the same. The details were as follows:

Composition of coating: the coating solution was an acetone-water (90:10) mixed solution of Opadry^{®} CA having a solid content of 8%.

### Preparation method:

### (1) Preparation of tablet core

Materials at prescribed doses were weighed, screened with 100 mesh sieve respectively, and mixed homogenously after sieving, and 95% ethanol solution was added to prepare soft materials. The soft materials were granulated through 30 mesh sieve, dried at 60°C for 1 h, and then sorted through 30 mesh sieve. Magnesium stearate was added and mixed homogenously. The mixture was tabletted by a single-punch tablet press machine with a punch of 10 mm and a hardness of 10 kgf to obtain a tablet core (the labeled amount of nicardipine hydrochloride was 80 mg).

### (2) Coating and perforation

Preparation of coating solution: 8% of coating powder (Opadry^{®} CA) and 92% of solution (containing 10% of water and 90% of acetone) were stirred with a stirring paddle for 4 h or more until the solution was homogeneous and clear and dusted.

Parameters for coating: hot air: 1200 rpm; exhaust air: 2500 rpm; coating temperature: 28°C; rotational speed of main engine: 10 rpm/min; flow rate: 8 ml/min; atomizing pressure: 0.2 MPa; spray gun pressure: 0.2 MPa.

Coating by pan-coating method: The average weight gain of the coating was 7.5%. The coating was dried and cured for 12 h in a constant temperature oven at 40°C. A drug-release hole with a hole diameter of 0.6 mm was punched on one side of the tablet using a laser perforator to obtain a osmotic pump controlled-release tablet of nicardipine hydrochloride with a specification of 80 mg.

Commercially available nicardipine hydrochloride sustained-release tablets (perdipine) were used as reference formulations for tests on *in vitro* release characteristics. Six tablets of each of formulations 1, formulations 2, and the reference formulations were taken and determined according to Approach 1 in General Principles 0931 of Preparations of *Chinese Pharmacopoeia (2015 Edition, Volume IV).* Water served as release medium. 5 ml of solutions were measured at 2, 4, 6, 8, 10, 12, 24 h respectively, filtered through a 0.45 µm filter membrane, while adding an equal volume of the release medium at the same temperature. An appropriate amount of filtrate was measured and detected according to the following conditions: Chromatographic conditions: chromatographic column: Kromasil C18 (250×4.6 mm, 5 µm), mobile phase containing 0.016 mol/L of buffer solution (pH6.8) of potassium hydrogen phosphate and potassium dihydrogen phosphate as an aqueous phase and methanol as an organic phase, aqueous phase:organic phase =28:72, detection wavelength: 236 nm, column temperature: 40°C, flow rate: 1.0 ml/min, sample size: 20 µL.

Preparation of control solution: an appropriate amount of nicardipine hydrochloride was weighed as a control. Methanol was added to dissolve it by ultrasound to prepare a control solution containing 50 µg of nicardipine hydrochloride per 1 ml.

Sample determination method: sample solutions and control solutions were precisely measured respectively for HPLC detection. The peak areas were recorded. The nicardipine hydrochloride content was calculated by an external standard method.

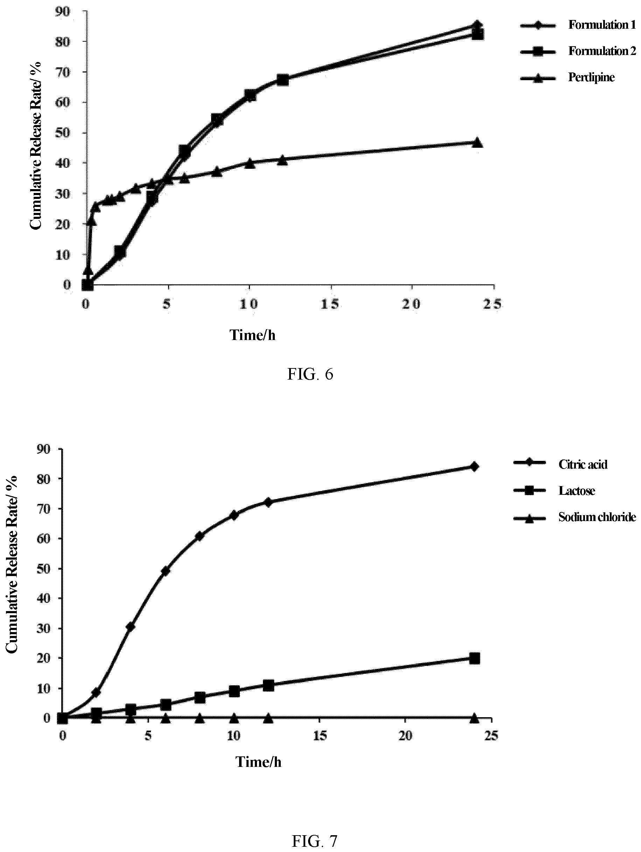

The cumulative release characteristics were calculated based on the sample content at each sampling point, and the release profile was plotted. The results were as shown in FIG. 6. The results showed that the drugs in both formulation 1 and formulation 2 were completely released over 24 h, and the drug release was steady within 12 h. Commercially available Nic sustained-release capsules had obvious burst release, and the release over 24 h was less than 50%. Compared with commercially available Nic sustained-release capsules, formulation 1 and formulation 2 showed complete release *in vitro* and exhibited good sustained-release characteristics.

### Example 5 Study on different penetration enhancers

The release characteristics of osmotic pump tablets in which the penetration enhancers were citric acid, lactose, and sodium chloride were studied. The microcrystalline cellulose and citric acid in the tablet core of formula 1 were replaced with the same weight of citric acid, lactose, and sodium chloride, and the other conditions were the same. Solid dispersions and osmotic pump tablets were prepared according to the preparation method for formula 1 in Example 4, and the cumulative release characteristics of the osmotic pump tablets at different points of time were determined respectively according to the method in Example 4. The results (FIG. 7) showed significant differences in release profiles of different penetration enhancers (accounting for 69.98% of the total weight of the tablet core). In the sodium chloride group, there was almost no release over 24 h; in the lactose group, the cumulative release characteristics over 24 h were about 20%; and in the citric acid group, the cumulative release characteristics over 24 h were greater than 80%.

### Example 6 Study on combinations with different penetration enhancers

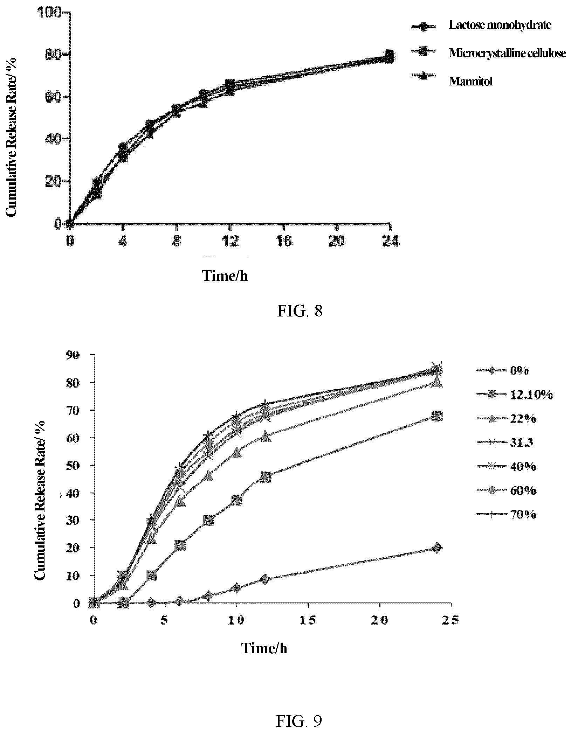

The release characteristics of osmotic pump tablets prepared by combining citric acid with different penetration enhancers were studied. The microcrystalline cellulose in the tablet core of formula 1 was replaced with the same weight of mannitol and lactose monohydrate, and the other conditions were the same. Solid dispersions and osmotic pump tablets were prepared according to the preparation method for formula 1 in Example 4, and the cumulative release characteristics of the osmotic pump tablets at different points of time were determined respectively according to the method in Example 4. The results (FIG. 8) showed similar release profiles of combinations with different penetration enhancers (accounting for 69.98% of the total weight of the tablet core).

### Example 7 Study on amount of penetration enhancer

According to the preparation method for formula 1 in Example 4, the amount of citric acid as a penetration enhancer was adjusted to 0%, 12.1%, 22%, 31.3%, 40%, 60%, and 69.98% of the weight of the tablet core, while keeping the total weight of the microcrystalline cellulose and citric acid constant and the weight of the other components unchanged. The release characteristics were determined according to the method in Example 4. The release profile was plotted as shown in FIG. 9. The results suggested that when the ratio of the citric acid as a penetration enhancer was 0%, the cumulative release characteristics of the prepared osmotic pump tablet were less than 80% over 0 to 24 h; when the ratio was 12.1%, the release characteristics of the osmotic pump tablet was greater than 65%; when the ratios were 22%, 31.3%, 40%, 60%, and 70%, the cumulative release characteristics of the prepared osmotic pump tablets were greater than 80% over 0 to 24 h. During the preparation, it was found that when the ratio of citric acid was 70%, the prepared soft material was more viscous, which was not conducive to preparation of wet particles.

### Example 8 Ratio of film-forming material to polyethylene glycol in coating material

Anhydrous citric acid was used as a carrier and the drug-carrier weight ratio was 1:0.19 to prepare a solid dispersion according to the preparation process in Example 1. The tablet core was prepared according to formula 1 in Example 4. The tablet core was coated according to the following coating composition:
Coating composition 1: the weight ratio of cellulose acetate to polyethylene glycol was 90:10, and 90% of aqueous acetone solution was used to formulate a coating solution.
Coating composition 2: the weight ratio of cellulose acetate to polyethylene glycol was 95:5, and 90% of aqueous acetone solution was used to formulate a coating solution.
Coating composition 3: the weight ratio of cellulose acetate to polyethylene glycol was 99:1, and 90% of aqueous acetone solution was used to formulate a coating solution.

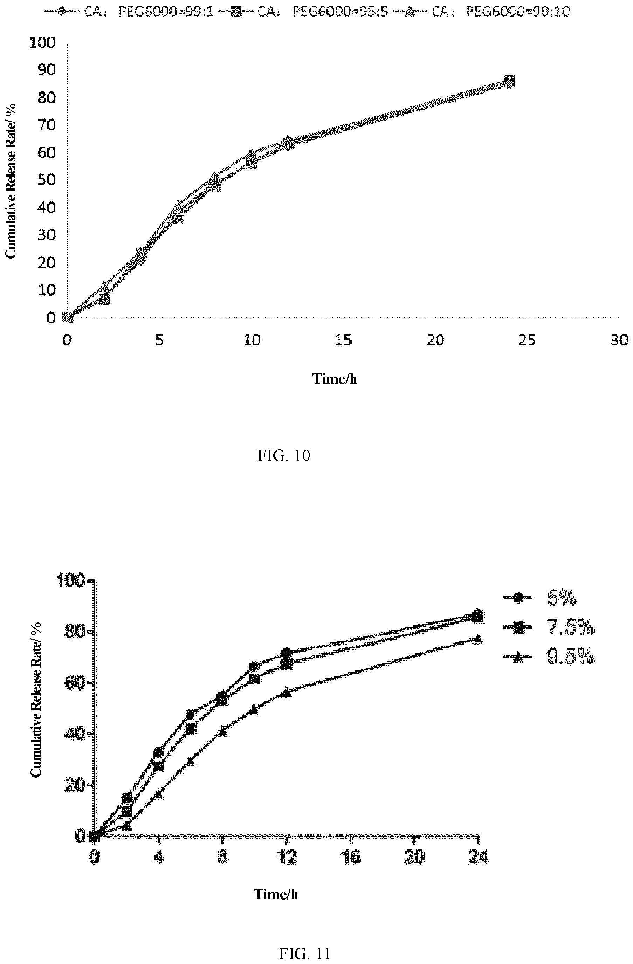

The weight gain of the coating was 7.5%. Tablets having different coating compositions were taken for test on release characteristics. The results (FIG. 10) suggested that the results of the cumulative release characteristics over 24 h of the osmotic pump tablets having different coating compositions were consistent and all greater than 80%.

### Example 9 Study on weight gain of coating

The tablet core of the osmotic pump tablet was prepared according to the method for formula 1 in Example 4. Afterwards, the tablet core of the osmotic pump was coated with the coating material Opadry^{®} CA. The mean weight gains were 5%, 7.5%, and 9.5%, respectively. The release characteristics were determined according to the method in Example 4 and the release profile was plotted.

The results (FIG. 11) suggested that the osmotic pump tablet with the coating weight gain of 9.5% had a cumulative release rate of approximate 80% over 0 to 24 h; the cumulative release rates over 24 h of the osmotic pump tablets with the coating weight gains of 5% and 7.5% were very close and both greater than 80%; and the release rate of the osmotic pump tablet with the coating weight gain of 7.5% over 0 to 24 h was more steady.

### Example 10 Study on stability

Formula 1 prepared in Example 4 was used and subjected to the influence factor test and acceleration test to study changes in the nicardipine hydrochloride content and related matters in the sample respectively and study the release characteristics at different sampling points of time under conditions for acceleration.

Influence factor test: Formulation 1 was taken and placed in a sealed clean container at 60°C for 10 days. Sampling was conducted on day 5 and day 10 to detect the content and related matters.

Formulation 1 was taken and placed in a constant-humidity closed container at 25°C under the condition of RH90±5%. Sampling was conducted on day 5 and day 10 to detect the content and related matters.

Formulation 1 was taken and placed in a light box under the illumination of 4500Lx±500Lx. Sampling was conducted on day 5 and day 10 to detect the content and related matters.

Acceleration test: Formulations 1 in three batches were taken, sealed and packaged, and placed at 40°C under drying conditions. Sampling was conducted in month 1, month 2, and month 3 respectively to detect the content, related matters, and release characteristics.

### Appearance: visual inspection.

Determination of content and related matter: Three tablets of formulation 1 were taken, and the coatings were removed. The tablets were ground. An appropriate amount of powder was precisely weighed (equivalent to about 25 mg of nicardipine hydrochloride), and placed in a 50 ml brown measuring flask, to which about 30 ml of methanol was added. Ultrasound was applied for 15 min to make the powder dispersed uniformly. The mixture was cooled to room temperature. The mobile phase was diluted to the scale, shaken well, and filtered. The filtrate was injected into samples to determine the related matters. 1 ml of the filtrate was precisely pipetted into a 10 ml brown measuring flask, diluted to the scale, and shaken well to obtain the desired product. Direct sampling was conducted to determine the Nic content.

Determination of release characteristics: The osmotic pump tablets were taken and determined according to Approach 1 in General Principles 0931 of Preparations of Chinese Pharmacopoeia (2015 Edition, Volume IV*)* using water, pH 2.0 solution and pH 4.0 solution as release media at 37°C in accordance with law. 5 ml of the solutions were measured at 2, 4, 6, 8, 10, 12, and 24 h respectively, filtered through a 0.45 µm microfiltration membrane, while adding an equivalent volume of the release media at the same temperature. The filtrate was measured and the absorption peak area at the wavelength of 236 nm was measured according to the high performance liquid chromatography (as shown below). The release characteristics of the nicardipine hydrochloride osmotic pump tablet were calculated by the external standard method.

Chromatographic conditions: chromatographic column: Kromasil C18 (250×4.6 mm, 5 µm), mobile phase containing 0.016 mol/L of buffer solution (pH6.8) of potassium hydrogen phosphate and potassium dihydrogen phosphate as an aqueous phase and methanol as an organic phase, aqueous phase:organic phase = 28:72, detection wavelength: 236 nm, column temperature: 40°C, flow rate: 1.0 ml/min, sample size: 20 µL.

Preparation of control solution: an appropriate amount of nicardipine hydrochloride as a control was weighed. Methanol was added to dissolve it by ultrasound to prepare a control solution containing 0.5 mg of nicardipine hydrochloride per 1 ml for determination of related matters. The above solution was diluted into a control solution containing 50 µg of nicardipine hydrochloride per 1 ml for determination of the content and release characteristics.

**Table 5 Determination results of the sample content and related matters**

| Sampling Time | Conditions | Sample Content % | Related Matter % |
|---|---|---|---|
| 0 | / | 96.22 | 0.111 |
| 5 | High temperature | 97.39 | 0.077 |
| | High humidity | 96.70 | 0.107 |
| | Light | 95.95 | 0.343 |
| 10 | High temperature | 95.19 | 0.083 |
| | High humidity | 96.76 | 0.041 |
| | Light | 95.60 | 0.042 |

The results showed that the drug content and related matters did not change significantly under the conditions of high temperature, high humidity, and light.

**Table 6 Acceleration test results**

| Sampling Time | Batch | Content % | Related Matter % | Release Characteristics % |
|---|---|---|---|---|
| Initial | 20170322 | 96.93 | 0.10 | 85.18 |
| | 170828-1 | 92.76 | 0.11 | 79.90 |
| | 170828-2 | 96.22 | 0.19 | 80.59 |
| 2 Months | 20170322 | 95.89 | 0.07 | 84.83 |
| | 170828-1 | 93.65 | 0.10 | 83.88 |
| | 170828-2 | 98.20 | 0.07 | 84.28 |
| 3 Months | 20170322 | 98.58 | 0.08 | 80.17 |
| | 170828-1 | 93.64 | 0.08 | 83.49 |
| | 170828-2 | 96.92 | 0.13 | 82.91 |

The above results showed that after 3 month acceleration, none of the drug content, related matters and release characteristics of formulation 1 changed significantly, and the quality was stable.

### Example 11 Test on pharmacokinetics

### Test Drug

Reference formulation: nicardipine hydrochloride sustained-release capsules (perdipine), 40 mg/capsule.

Test formulation: nicardipine hydrochloride osmotic pump tablets (NicT, formulation 1), 80 mg/tablet.

### Test Animal

Six healthy beagles (half male and half female) each weighed 7 to 10 kg were divided into two groups, three in Group A (two males and one female) and three in Group B (two females and one male).

### Administration and Blood Sampling

No drugs were administered to beagles for three weeks. They fasted for 12 h before administration and weighed early in the morning. Blank blood was collected before administration:
One capsule of perdipine was administered in Group A. One tablet (80 mg) of NicT was administered in Group B with 30 ml of water, and care was taken to keep the integrity of the osmotic pump tablet. 2 ml of blood was collected in a centrifuge tube coated with heparin sodium from Group A at 0.25, 0.5, 1, 1.5, 2, 2.5, 3, 4, 6, 8, 10, 12, and 24 h after the administration respectively, and centrifuged at 3000 r/min for 10 min. The plasma was separated and cryopreserved at -70°C. In Group B, the blood was collected at 1, 2, 3, 4, 5, 6, 7, 8, 10, 12, and 24 h after the administration. One week after the cleaning period, cross-administration of drugs was carried out (one tablet (80 mg) of NicT was administered in Group A, and one capsule of perdipine was administered in Group B).

### Pretreatment of Plasma Sample

0.2 ml of plasma was transferred into a 1.5 ml centrifuge tube with a plug, to which 20 µl of internal standard solution (1.25 µg/ml) was added, and vortexed for 40 s. 0.8 ml of tert-butyl methyl ether was added, vortexed for 4 min to make them mixed homogeneously, and then centrifuged at 12000 rpm×10 min. The organic layer was transferred into another 1.5 ml centrifuge tube, concentrated by centrifuging at 25°C until evaporation drying, re-dissolved in 100 µl of mobile phase, and centrifuged at a high speed of 12000 rpm×10 min after homogeneous mixing by vortex. The supernatant was directly injected into the sample. High Performance Liquid Chromatography-Mass Spectrometry (HPLC-MS/MS) technology was adopted for determination.

### HPLC-MS/MS Determination

Chromatographic conditions: the chromatographic column was Agilent Eclipse XDB-C18 (4.6×150 mm, 5 µm); the methanol:0.1% formic acid solution was a mobile phase; gradient elusion was adopted at a flow rate of 0.6 ml/min; the gradients were listed in the table below; Stop Time: 7 min; Post Time: 6 min; column temperature 50°C; injection volume: 5 µl.

**Table 7 Elution conditions of chromatographic column**

| Time (min) | Methanol (%) | 0.1% Aqueous Formic Acid Solution (%) |
|---|---|---|
| 0 | 70 | 30 |
| 4 | 95 | 5 |
| 6 | 95 | 5 |
| 6.01 | 70 | 30 |

Mass Spectrum conditions: ESI+ion source were used; the flow rate of the dry gas was 9 L/min; the temperature of the dry gas was 300°C; the capillary voltage was 5500 V MRM operating mode was selected for primary/secondary mass spectrometry. The detection ions used for quantitative and qualitative analysis were as follows: F=140, CE=20, nicardipine hydrochloride [M+H]+ m/z 480.2→315.1 (quantitative), m/z 480.2→148.0 (qualitative); F=80, CE=20, internal standard nimodipine [M+H]+ m/z419.0→301.1 (quantitative), m/z 419.0→343.0 (qualitative).

### Test Results

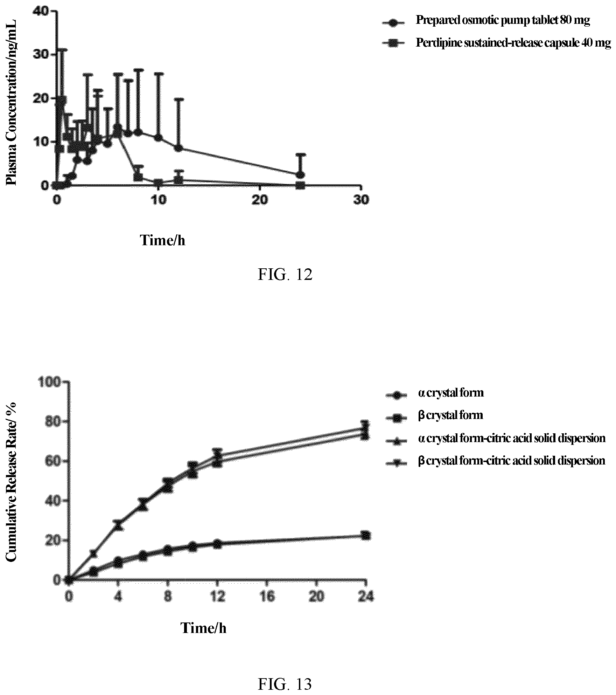

A drug-time curve was plotted based on the measured plasma concentration (FIG. 12). The results showed that the plasma concentration of perdipine in beagle fluctuated between 0 and 20 ng/ml, showing a bimodal curve; the plasma concentration of formulation 1 (80 mg) fluctuated between 0 and 15 ng/ml, showing a unimodal curve, and the plasma concentration was relatively steady. Hence, perdipine had a controlled-release effect within 2 to 10 h in the beagle.

The main pharmacokinetic parameters, calculated by statistical moment method, were shown in Table 8.

**Table 8 Pharmacokinetic parameters for Nic at different doses**

| Samples | Dose (mg) | Cmax (ng/ml) | Tmax (h) | AUC (0→t) (ng/ml*h) | AUC (0→∞) (ng/ml*h) | MRT (0→4) (h) |
|---|---|---|---|---|---|---|
| Perdipine | 40 | 19.66 | 0.5 | 84.045 | 84.559 | 3.91 |
| Formulation 1 | 80 | 13.42 | 6 | 219.01 | 299.257 | 8.06 |

The data showed that compared with the commercially available perdipine sustained-release capsules, formulation 1 had a delayed peak time Tmax (from 1/3 h to around 7 h) in beagle, a relatively steady plasma concentration, significantly prolonged mean residence time MRT, and exhibited controlled-release characteristics. The bioavailability was significantly improved.

### Comparative Example 1 Comparison between release characteristics of osmotic pump tablets prepared from Nic solid dispersion and from Nic raw material

Nicardipine hydrochloride (α and β crystal forms) was used as a raw material to prepare osmotic pumps (Group 1 and Group 2) according to the following formulae. At the same time, nicardipine hydrochloride (α and β crystal forms) and anhydrous citric acid were used at a drug-loading ratio (w/w) of 1:0.37 to prepare Nic/citric acid solid dispersions according to the method of Example 1, and then to prepare osmotic pumps (Group 3 and Group 4) according to the following formulae. The profiles of the release characteristics of the samples in the four groups were determined according to the method of Example 4 and were as shown in FIG. 13.

**Table 9 Formulae in Comparative Example**

| Raw and Auxiliary Materials Composition | Prescribed Amount (mg/Tablet) | | | | | | | |
|---|---|---|---|---|---|---|---|---|
| | Group 1 | | Group 2 | | Group 3 | | Group 4 | |
| Active ingredient | α-Form API | 80 | β-Form API | 80 | α-Nic/citric acid Solid dispersion | 110 | β-Nic/citric acid Solid dispersion | 110 |
| Penetration enhancer | Citric acid | 160 | Citric acid | 160 | Citric acid | 130 | Citric acid | 130 |
| Diluent | Lactose monohydrate | 160 | Lactose monohydrate | 160 | Lactose monohydrate | 160 | Lactose monohydrate | 160 |
| Lubricant | Magnesium stearate | 3 | Magnesium stearate | 3 | Magnesium stearate | 3 | Magnesium stearate | 3 |
| Adhesive | PVPk30 | 6 | PVPk30 | 6 | PVPk30 | 6 | PVPk30 | 6 |
| Disintegrant | PVPP | 6 | PVPP | 6 | PVPP | 6 | PVPP | 6 |
| Coating material | Opadry^{®} CA | | Opadry^{®} CA | | Opadry^{®} CA | | Opadry^{®} CA | |
| Weight gain of coating | 7% to 7.5% | | 7% to 7.5% | | 7% to 7.5% | | 7% to 7.5% | |

The results of FIG. 13 showed that the release characteristics of common single-layer osmotic pump tablets prepared from α- and β-form raw materials were all less than 25%, but the release characteristics of the improved single-layer osmotic pump tablets prepared from the corresponding citric acid solid dispersions as intermediate carriers reached 80%.

The present disclosure is described above with reference to the preferred embodiments despite the fact that these embodiments are just exemplary and for illustrative purpose only. On that basis, various substitutions and modifications may be made to the present disclosure, all of which fall within the scope of protection for the present disclosure.

## Claims

1. An osmotic pump controlled-release tablet of an insoluble drug, wherein the osmotic pump controlled-release tablet comprises a tablet core, a semi-permeable membrane coating, and a drug-release hole, the tablet core comprises a solid dispersion of the insoluble drug and a penetration enhancer, and the solid dispersion of the insoluble drug comprises the insoluble drug and a carrier;
the insoluble drug is selected from the group consisting of nicardipine, nifedipine, felodipine, and pharmaceutically acceptable salts thereof, and the carrier is selected from the group consisting of organic acids.

2. The osmotic pump controlled-release tablet of the insoluble drug according to claim 1, wherein the penetration enhancer is an organic acid or a combination of an organic acid with any one or more of sodium chloride, mannitol, and lactose.

3. The osmotic pump controlled-release tablet of the insoluble drug according to claim 1 or 2, wherein the organic acids are citric acid, fumaric acid, succinic acid, tartaric acid, cholic acid, lecithin or deoxycholic acid.

4. The osmotic pump controlled-release tablet of the insoluble drug according to claim 3, wherein the organic acids are citric acid, fumaric acid or succinic acid.

5. The osmotic pump controlled-release tablet of the insoluble drug according to claim 3, wherein the organic acids are citric acid.

6. The osmotic pump controlled-release tablet of the insoluble drug according to claim 1, wherein the penetration enhancer is citric acid.

7. The osmotic pump controlled-release tablet of the insoluble drug according to claim 1, wherein the insoluble drug is nicardipine.

8. The osmotic pump controlled-release tablet of the insoluble drug according to claim 7, wherein the insoluble drug is α-crystal-form nicardipine, β-crystal-form nicardipine or a mixed crystal thereof.

9. The osmotic pump controlled-release tablet of the insoluble drug according to any one of claims 1, 7, and 8, wherein a weight ratio of the insoluble drug to the carrier in the solid dispersion is 1:(0.1-1).

10. The osmotic pump controlled-release tablet of the insoluble drug according to claim 9, wherein the weight ratio of the insoluble drug to the carrier in the solid dispersion is 1 :(0.1-0.8).

11. The osmotic pump controlled-release tablet of the insoluble drug according to claim 9, wherein the weight ratio of the insoluble drug to the carrier in the solid dispersion is 1 :(0.15-0.6).

12. The osmotic pump controlled-release tablet of the insoluble drug according to any one of claims 1 to 6, wherein the penetration enhancer accounts for 10% to 70% of a total weight of the tablet core.

13. The osmotic pump controlled-release tablet of the insoluble drug according to claim 12, wherein the penetration enhancer accounts for 20% to 60% of the total weight of the tablet core.

14. A solid dispersion of an insoluble drug, comprising an insoluble drug and a carrier material;
wherein the insoluble drug is selected from the group consisting of nicardipine, nifedipine, felodipine, and pharmaceutically acceptable salts thereof, and the carrier material is selected from the group consisting of organic acids.

15. The solid dispersion of the insoluble drug according to claim 14, wherein the organic acids are citric acid, fumaric acid, succinic acid, tartaric acid, cholic acid, lecithin or deoxycholic acid.

16. The solid dispersion of the insoluble drug according to claim 14, wherein the organic acids are citric acid, fumaric acid or succinic acid.

17. The solid dispersion of the insoluble drug according to claim 14, wherein the organic acids are citric acid.

18. A method for preparing an osmotic pump controlled-release tablet of an insoluble drug according to any one of claims 1 to 13, comprising steps of:
a) preparing a solid dispersion of the insoluble drug by a solvent method;
b) mixing the solid dispersion with a penetration enhancer, and optionally adding an appropriate amount of an additional excipient;
c) directly tablet pressing or pressing to form a tablet core after granulation; and
d) externally covering with a semi-permeable membrane and perforating by laser or mechanically.

19. The method for preparing the osmotic pump controlled-release tablet of the insoluble drug according to claim 18, wherein the step of the solvent method comprises: dissolving the drug and a carrier in an organic solvent, removing the organic solvent under reduced pressure until foaming, and subjecting to rotary evaporation, sieving, and vacuum drying to obtain the solid dispersion of the insoluble drug;
the organic solvent is selected from the group consisting of ethanol, methanol, and tert-butanol.

20. The method for preparing the osmotic pump controlled-release tablet of the insoluble drug according to claim 19, wherein the organic solvent is methanol.

21. The method for preparing the osmotic pump controlled-release tablet of the insoluble drug according to claim 18, wherein the step of the solvent method comprises: dissolving the drug and a carrier in an organic solvent, removing the organic solvent by spray drying, and subjecting to vacuum drying to obtain the solid dispersion of the insoluble drug;
the organic solvent is selected from the group consisting of ethanol, methanol, and tert-butanol.

22. The method for preparing the osmotic pump controlled-release tablet of the insoluble drug according to claim 21, wherein the organic solvent is methanol.

23. The method for preparing the osmotic pump controlled-release tablet of the insoluble drug according to claim 18, wherein the step of the solvent method comprises: dissolving the drug and a carrier in an organic solvent, and removing the organic solvent by a freeze drying method to obtain the solid dispersion of the insoluble drug, and
the organic solvent is tert-butanol.
